(19) Europäisches Patentamt
European Patent Office
Office européen des brevets

(11) Publication number: 0 158 720
A1

(12) # EUROPEAN PATENT APPLICATION

(21) Application number: 84114574.1

(22) Date of filing: 30.11.84

(51) Int. Cl.[4]: C 07 C 51/353
C 07 C 53/08
//B01J23/58

(30) Priority: 02.12.83 US 557273

(43) Date of publication of application:
23.10.85 Bulletin 85/43

(84) Designated Contracting States:
DE FR GB IT NL

(71) Applicant: UNION CARBIDE CORPORATION
Old Ridgebury Road
Danbury Connecticut 06817(US)

(72) Inventor: Schreck, David James
5226 Sun Valley Drive
Cross Lanes (25313)(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) **Synergistic production of carboxylic acids from organic formate esters.**

(57) A process for the production of organic carboxylic acids by the catalytic reaction of organic formate esters in contact with a homogeneous catalyst system containing rhodium metal atom and a mixture of lithium iodide and methyl iodide.

EP 0 158 720 A1

# SYNERGISTIC PRODUCTION OF CARBOXYLIC ACIDS FROM ORGANIC FORMATE ESTERS

## BACKGROUND OF THE INVENTION

The production of organic compounds using carbon monoxide or synthesis gas, which is a mixture of carbon monoxide and hydrogen, as reactant has been known for a significant period of time. It is well known that one can produce methanol directly from synthesis gas and that methanol can be further reacted by hydroformylation, homologation and carbonylation reactions to produce acetaldehyde, ethanol and acetic acid or its methyl ester, respectively. It is also known that esters, ethers, and other organic compounds can be reacted with carbon monoxide or synthesis gas to produce oxygenated organic compounds. The difficulties, however, have resided in the ability to carry out any one of these chosen reactions to produce the desired compound at acceptable efficiency, conversion rate and selectivity.

In almost all instances the reaction is generally catalyzed using Group VIII transition metal compound as catalyst and a halogen as the promoter. It is known that many other metal compounds and promoters can be used. In addition, the prior art has disclosed the use of secondary activators or ligands in conjunction with the metal catalysts and promoters. These secondary activators can be other metallic salts or compounds, amines, phosphorus compounds, as well as a multitude of

other compounds that have been disclosed in the published literature. Thus, a typical catalyst system contains the metal atom catalyst, promoter and, optionally, ligands, solvents and secondary activators. Though a significant amount of literature does exist describing the production of acetic acid by the isomerizationof methyl formate, to our knowledge it does not disclose or suggest our invention. Several of the pertinent patents in this area are discussed below.

French Patent No. 2,317,269

discloses the production of aliphatic carboxylic acids by the reaction of an alcohol with carbon monoxide in the presence of a catalyst containing at least three essential components, iridium atom, copper atom and halogen. This is not our process.

In European Patent Application No. 0018927

there is described a process for the production of monocarboxylic acids by the carbonylation of an alcohol using a nickel catalyst, a halide and a solvent; in this reference synthesis gas is used. In the process of the invention of this instant application an organic acid is produced from a formate ester using a rhodium atom catalyst and lithium iodide in conjunction with methyl iodide.

In European Patent Application No. 0045637

there is disclosed the direct

conversion of formic acid esters to their corresponding carboxylic acids without the presence of carbon monoxide using as catalyst a soluble irridium salt and an iodine promoter. This is not our catalytic process.

Another known procedure for producing acetic acid is the catalytic isomerization of methyl formate as shown by the reaction:

$$CH_3OOCH \longrightarrow CH_3COOH$$

This procedure is shown in U.S. 1,697,109.

The process described is a vapor phase isomerization reaction carried out at 200°C to 450°C at a pressure up to 200 atmospheres using a metal oxide or acetate catalyst. It does not disclose the use of rhodium and lithium iodide plus methyl iodide.

U.S. 2,508,513

claims an iron metal atom based catalyst, e.g. nickel, promoted with methyl iodide for the isomerization of methyl formate to acetic acid, carried out at 300°C to 400°C and a pressure up to 400 atmospheres. Carbon monoxide may be present. It does not disclose the use of rhodium and lithium iodide plus methyl iodide.

U.S. 3,060,233

discloses the carbonylation of methanol to acetic acid using a metal of the iron group of the Periodic Table and a halide. It does not disclose use of rhodium or the use of a formate ester.

U.S. 3,769,329

discloses the production of carboxylic

acids from alcohols, or the ester, ether and halide derivatives thereof, and carbon monoxide using a rhodium catalyst and a halogen component. It does not mention mixtures of lithium iodide and methyl iodide.

U.S. 3,798,267

relates to the conversion of methyl formate to acetic acid in the presence of a catalyst system consisting essentially of activated carbon and a halogen promoter. The reference uses catalyst and starting materials different than those employed in the invention of this application.

U.S. 4,194,056

discloses the production of carboxylic acid from methyl formate using a soluble rhodium catalyst, halogen promoter and carbon monoxide. This is not the process of the instant invention, nor does this reference suggest or disclose the use of a mixture of lithium iodide plus methyl iodide and the unexpected results achieved by its use.

U.S. Patent No. 4,212,989

describes a process for producing carboxylic acids or their esters by reacting an alcohol or an ether with carbon monoxide using a Group VIII metal catalyst and an iodine promoter. The reference contains no suggestion or disclosure of the production of organic carboxylic acids from a formate ester.

British Patent Specification 1,286,224

relates to the reaction of methyl formate with carbon

monoxide in contact with a rhodium catalyst and a halogen promoter to produce acetic acid. It contains no recognition of the distinct advantages achieved with the use of lithium iodide plus methyl iodide, in fact it does not mention mixtures of these specific compounds.

British Patent Specification 1,293,193

relates to the direct conversion of formic acid esters to the corresponding carboxylic acids, in the presence of carbon monoxide, a catalyst that is a Group IIb or VIII metal and an organic polar solvent. It does not disclose use of rhodium atom plus lithium iodide and methyl iodide.

Japanese Patent Publication 50-16773

discloses the production of an organic acid from the corresponding formic acid ester in the presence of carbon monoxide using a catalyst system containing cobalt, iron or mercury and a halogen plus an alkali metal salt of a lower aliphatic carboxylic acid, triamine or cyclic amine.

Japanese Patent Publication 51-65703,

discloses the reaction of methyl formate in the presence of carbon monoxide using a system containing a rhenium catalyst and halogen compound to produce acetic acid.

Japanese Patent Publication 56-22745

discloses the isomerization of a formic acid ester to the corresponding acid in the presence of carbon monoxide, palladium atom, halogen and base.

Japanese Patent Application No. 56-73040

relates to a process for producing acetic acid by isomerizing methyl formate in the presence of carbon monoxide using a nickel catalyst, an iodine compound and an organic nitrogen compound.

Japanese Patent Application 56-83439

discloses a method for producing acetic acid by heating methyl formate and carbon monoxide in contact with a catalyst containing palladium, ruthenium and/or irridium metal atom and a halide promoter.

None of the five Japanese Patent Applications disclose a process for producing acetic acid from a formate ester using a catalyst mixture consisting essentially of rhodium metal atom and lithium iodide plus methyl iodide.

It can be seen that the prior art contains many disclosures dealing with the catalytic production of acetic acid, including its production by the isomerization of methyl formate. The art also discloses the production of other organic carboxylic acids by the isomerization of other formate esters. One of the disadvantages in many of these references is the presence of water with the eventual need to remove it from the desired organic acid product. This removal is both complicated and costly. Other disadvantages often include the simultaneous occurrence of other reactions leading to the formation of by-products, such as, dimethyl acetal, methyl acetate, and ethanol. These

reactions compete with the organic acid production resulting in low conversion rate and selectivity to organic acid.

Many processes employed for the production of organic acids use a catalyst system containing a source of metal atom and a source of halide atom. The alkali metal halides are often mentioned as suitable halide sources, but no distinction is made between any specific one of the alkali metal halides or between any other halogen compound. Nor do any of the references suggest or recognize the synergistic advantage of the use of mixtures of lithium iodide and methyl iodide in conjunction with rhodium catalyst.

## SUMMARY OF THE INVENTION

A catalyst system and process for the production of organic acids at high efficiency, selectivity and conversion rate by the reaction of organic esters of formic acid, such as methyl formate, in the presence of carbon monoxide or of synthesis gas has been found. The catalyst system charged to the reactor in our process contains rhodium atoms, lithium iodide and methyl iodide and optionally an organic ligand. The use of mixtures of lithium iodide and methyl iodide in this system within the ranges defined results in a synergistic effect with unexpectedly high efficiency, high conversion rate or activity and high selectivity not heretofore achieved.

## DESCRIPTION OF THE INVENTION

In the catalytic reactions of synthesis gas or carbon monoxide in processes to produce

oxygenated organic compounds there are several criteria required of the catalyst. The catalyst must be as stable as possible, it should have a high activity or conversion rate, and it should have as high a selectivity for the desired product as possible.

Stability of the catalyst relates to how long the catalyst remains functional before either breaking down or losing its catalytic effect.

Activity or conversion rate relates to the amounts of reactants the catalyst converts to product per unit of time, generally expressed in g. mole per liter per hour (g mole/l/hr).

Selectivity relates to the quantity of desired product produced, generally expressed in mole percent, based on the total amount of both desired products and undesired products produced.

The goal to be achieved is high values for all three criteria and continued efforts are being made to find new catalyst compositions to reach this goal without having a significant detrimental effect on the overall process. Toward this goal the prior art has developed catalyst systems containing a wide variety of metal atoms, promoters and activators, in many cases with diverse other components added. Though these catalyst systems are effective, improvement is always desirable.

The present invention is based on the unexpected and unpredictable discovery that the rhodium-lithium iodide plus methyl iodide system is an unexpectedly superior catalytic system showing a synergistic effect for the production of organic

acids from esters of formic acid (hereinafter "formates") at unexpected high efficiency, selectivity and conversion rate. It was also found that a ligand, $ER''_3$, can also be present as an optional component of the system. This unexpected synergistic improvement in efficiency, selectivity and conversion rate is achieved when the rhodium catalyzed system's components are maintained within a defined range and when both lithium iodide plus methyl iodide are present as the source of the halogen component in the system. Optionally a solvent and/or diluent can also be present. The improved catalyst system of this invention can be portrayed as containing the components $Rh-LiI-CH_3I-ER''_3$, wherein Rh is the rhodium containing compound and $ER''_3$ is optionally present.

In the process of our invention formates are reacted with carbon monoxide using a particular catalyst system containing rhodium atoms and both lithium iodide and methyl iodide. This system synergistically produces commercially desirable organic acids at unexpectedly high efficiency, conversion rate and selectivity, with a minimum of by-products and without the presence of water. The overall reaction that occurs is theoretically:

$$HCOOR \longrightarrow RCOOH$$

In the above formula R is a monovalent hydrocarbyl group. It can be an alkyl group having from 1 to 30 carbon atoms, preferably from 1 to 15 carbon atoms, and most preferably from 1 to 5 carbon atoms; an alkenyl group having from 2 to 30 carbon

atoms, preferably from 2 to 15 carbon atoms and most preferably from 2 to 5 carbon atoms; or an aryl, aralkyl or alkaryl group having 6 or 10 ring carbon atoms, e.g., phenyl and naphthyl, with from 1 to 10 carbon atoms, preferably 1 to 4 carbon atoms, in the alk-moiety thereof. The R group can be linear or branched and it can be unsubstituted or substituted with groups which will not have an adverse effect on the reaction; further; the alkenyl groups can contain more than one unsaturated bond.

Illustrative of suitable formate esters one can mention methyl formate, ethyl formate, the propyl formates, the butyl formates, the decyl formates, 2-ethylhexyl formate, stearyl formate, phenyl formate, benzyl formate, vinyl formate, allyl formate, naphthyl formate, tolyl formate.

The rhodium component of the catalyst system can be supplied from any number of sources, many of these are known to those of ordinary skill in the art. Thus, it is not necessary for an understanding thereof to specifically enumerate every suitable type and every specific compound since any of the known rhodium compounds can be used.

The essential rhodium component of the catalyst system of the present invention may be provided by introducing into the reaction zone a compound of rhodium or may be provided by introducing into the reaction zone rhodium. Among the materials which may be charged to the reaction zone to provide the rhodium component of the catalyst system of the present invention are rhodium metal, rhodium salts and oxides, organo rhodium

compounds and coordination compounds of rhodium,

Specific examples of materials capable of providing the rhodium constituent of the catalyst system of the present invention may be taken from the following list of suitable materials.

$RhCl_2$

$RhBr_3$

$RhI_2$

$RhCl_3$ $3H_2O$

$RhBr_3$ $3H_2O$

$Rh_2(CO)_4Cl_2$

$Rh_2(CO)_4Br_2$

$Rh_2(CO)_4I_2$

$Rh_2(CO)_8$

$Rh[(C_6H_5)_3P]_2(CO)I$

$Rh[(C_6H_5)_3P]_2(CO)Cl$

Rh metal

$Rh(NO_3)_3$

$RhCl[(C_6H_5)_3P]_2(CH_3I)_2$

$Rh(SnCl_3)[(C_6H_5)_3P]_2$

$RhCl(CO)[C_6H_5)_3As]_2$

$RhI(CO)[(C_6H_5)_3Sb]_2$

$[(n-C_4H_9)_4N][Rh(CO)_2X_2]$where X=Cl-, Br-, I-

$[(n-C_4H_9)_4AS]_2[Rh(CO)_2Y_4]$where X=Br-, I-

$[(n-C_4H_9)_4P][Rh(CO)I_4]$

$Rh[(C_6H_5)_3P]_2(CO)Br$

$Rh[(n-C_4H_9)_3P]_2(CO)Br$

$Rh[(n-C_4H_9)_3P]_2(CO)I$

$RhBr[(C_6H_5)_3P]_3$

$RhI[(C_6H_5)_3P]_3$

$RhCl[C_6H_5)_3P]_2$

$RhCl[(C_6H_5)_3P]_3H_2$

$[(C_6H_5)_3P]_3Rh(CO)H$

$Rh_2O_3$

$[Rh(C_3H_4)_2Cl]_2$

$K_4Rh_2Cl_2(SnCl_2)_4$

$K_4Rh_2Br_2(SnBr_3)_4$

$K_4Rh_2I_2(SnI_2)_4$

In addition, one can use the other Group VIII transition metals comprising the iron triad, i.e., iron, ruthenium, osmium; the cobalt triad, i.e., cobalt, rhodium, iridium; or the nickel triad, i.e., nickel, palladium, platinum. Though these will catalyze the reaction, the preferred metals are nickel and rhodium, with the most preferred being rhodium.

The rhodium or Group VIII metal atom concentration can vary over a wide range. Enough metal atom must be present to achieve reasonable reaction rates; however, an excess may on occasion result in undesired by-products formation. For simplicity, the rhodium atom will be used in the specification with the understanding that it also applies to the other transition metals of Group VIII. The mole ratio of rhodium to formate can vary from 1:25 to 1:40,000, the preferred range is from 1:40 to 1:5,000, with the most preferred range being from 1:100 to 1:2,000. The amount used is not a critical feature in this invention and higher rhodium concentrations are acceptable but are influenced by economic considerations.

The second component of the catalyst system is lithium iodide. It can be charged directly, or

it can be formed in situ by any combination of lithium compound and iodine component that will result in the formation of lithium iodide during the reaction. Lithium bromide can also be used but the iodide is preferred. The presence of lithium iodide or lithium bromide in conjunction with methyl iodide is a critical feature of this invention. Direct charge of lithium iodide is the preferred form. However, any convenient combination of compounds for in situ formation of lithium iodide can be used. This includes the use of lithium carboxylates, carbonates and the like with a halogen compound such as iodine or an alkyl halide. A suitable combination for in situ formation is lithium carboxylate and an alkyl halide.

The third essential component of the system is methyl iodide, which can be added directly or formed in situ by the use of hydrogen iodide, which reacts to produce methyl iodide. The Rh:$CH_3I$ mole ratio can vary from 1:1 to 1:1,000, preferably from 1:2 to 1:450, and most preferably from 1:8to 1:150.

Sufficient lithium iodide and methyl iodide must be present to exert a promoting effect on the reaction and to result in high efficiency, conversion rate and selectivity to the corresponding organic acid. The mole ratio of Rh:LiI can vary over a wide range. A Rh:LiI mole ratio of from 1:1 to 1:1000 can be employed, the preferred range is from 1:2 to 1:450 and most preferably it is from 1:8 to 1:150. The mole ratio of LiI to $CH_3I$ can vary from 1:1,000 to 1,000:1, preferably from 1:450 to 450:1, and most preferably from 1:150 to 150:1.

As indicated, an organic ligand of the general formula $ER_3''$ can optionally be present in the reaction system. The use of such ligands is known, as are their identities, to those skilled in this art. In this formula E represents a Group VA element, e.g., N, P, As, Sb and Bi, and R'' represents an organic moiety. The ligand can serve as a catalyst stabilizer and/or to further enhance efficiency, conversion rate and selectivity, especially when the reaction is carried out at higher temperatures, for example at about 200°C or above. The ligand also serves to inhibit equipment corrosion in some instances. However, the use of a ligand is not mandatory and the reaction can be carried out without it.

A large number of organic ligands is known and any of these can be used provided they do not have an adverse effect on the reaction. Among those of particular utility are the tertiary amines and the tri- and pentavalent phosphorus compounds. Though those skilled in the art know these compounds, illustrative of suitable compounds one can mention triethylphosphine, tributylphosphine, tri-2-ethylhexylphosphine, triphenylphosphine, tri(4-methoxyphenyl)phosphine, tri-p-tolylphosphine, tri(3-chlorophenyl)phosphine, diphenyl hexylphosphine, dimethyl (3-methoxyphenyl)phosphine, dibutyl stearylphosphine, tribenzylphosphine, dipropyl phenylphosphine, ethyl dipropylphosphine, tricyclohexylphosphine, cyclohexyl dibutylphosphine, propyl diphenylphosphine, dipropyl phenylphosphine, phenyl diethylphosphine, tridecylphosphine,

trioctadecylphosphine, tribenzylphosphine, methyl diethylphosphine, ethyl diphenylphosphine, tolyl diethylphosphine, cyclohexyl diethylphosphine, diethyl cyclohexylphosphine, bis-(diphenylphosphino)-ethane, bis-(diethylphosphino)-propane, bis-(di-phenylphosphino)-butane, bis-(diethylphosphino)-octane, trimethylamine, triethylamine, tri-n-butylamine, tri-t-butylamine, tri-2-ethylhexylamine, methyl dibutylamine, tridodecylamine, tristearylamine, ethyl dibutylamine, tricyclohexylamine, triphenylamine, tri(4-methoxyphenyl)amine, tri(p-chlorophenyl)-amine, dibutyl phenylamine, dipentyl cyclopentylamine, ethyl diphenylamine, trinaphthylamine, tri-p-tolylamine, tri-benzylamine, tri(3-methylcyclohexyl)amine, and the arsines, stibines and bismuthines corresponding to the above-identified phosphines and amines.

They can be used singly or, if one desires, mixtures containing 2 or more ligands can be used. One can also employ a phosphine oxide or phosphite corresponding to the above phosphines as the ligand; these are also well known.

The concentration of ligand charged can vary from a molar ratio of ligand to rhodium of from 50:1 to 1:50, preferably from 10:1 to 1:10, most preferably 3:1 to 1:1.

In addition to the ligand one can optionally have a solvent present. Many essentially inert solvents are known as useful, essentially inert, diluents and illustrative thereof one can

mention 1,4-dioxane, the polyethylene glycols di-ethers or esters, diphenyl ether, sulfolane, toluene, carboxylic acids as well as any other diluent or solvent which does not interfere with the reaction to any significant extent. The reaction is preferably carried out in the absence of any solvent or diluent other than those required to introduce reactants or catalyst components.

The reaction is carried out at a temperature of from 50°C to 350°C, preferably from 120°C to 220°C and most preferably from 140°C to 200°C. When the reaction is carried out at temperatures above 200°C in the presence of an $ER''_3$ ligand, the phosphines are the preferred ligands.

The pressure of the reaction can be from 10,35 to 690 bar (150 psig to 10,000 psig), preferably from 13,8 to 69 bar (200 psig to 1,000 psig), most preferably from (200 psig to 500 psig) 13,8 to 34,5 bar.

The reaction time varies depending upon the reaction parameters, reactor size and charge, and the individual components employed at the specific process conditions. The reaction can be a batch or continuous reaction.

The synergistic effect of mixtures of lithium iodide and methyl iodide on conversion rate was completely unexpected and unpredictable. Significant rate increases were obtained as compared to the use of rhodium with lithium iodide alone or rhodium with methyl iodide alone. The significantly enhanced reaction rates in the production of organic acids from formate esters are very advantageous in

that they result in increased productivity from an available reactor, or they would allow for a significant reduction in size for a new reactor. Another advantage is that equivalent productivity can be achieved with the use of much less of the expensive rhodium catalyst. Use of the system of this invention results in production of acetic acid from methyl formate at typical conversion rates of from 10 to 50 gmoles/l·h and typical selectivities of from 90% to 99% at 180°C and 27.6 bar (400 psig) CO pressure. The values obtained exceed those obtained when either methyl iodide or lithium iodide were used individually with rhodium.

The experiments and examples detailed below were carried out in a Hasteloy® steel autoclave reactor having a volume of 300 ml, which was equipped with temperature and pressure sensing means, heating and cooling means, agitator and inlet and outlet means for introducing and removing components from the reactor. The autoclaves used in synthesis gas reactions are well known in the art and can be used in this process.

Prior to charging the reactants the autoclave was washed with methanol at 100°C under a nitrogen gas pressure of 34.5 to 69 bar (500 to 1,000 psig) by agitating for 30 minutes. The autoclave was drained, rinsed with dry acetone, and dried with nitrogen. The liquid components were charged to the cleaned autoclave first and then the solid components were added and stirred. The autoclave was closed and purged with carbon monoxide and then pressurized to the desired pressure with carbon

monoxide. The autoclave contents were heated to the selected temperature, with agitation (usually 750 rpm), in about 45 minutes. After the desired temperature was reached, the reaction was allowed to consume gas for the time period indicated. During this time the pressure was maintained by addition of carbon monoxide as needed.

At the end of the reactor run, the contents were cooled, generally to about 10°C. A vapor phase sample was taken for gas chromatography analysis; the gas phase was vented through 2 dry-ice acetone traps and then through a 10 liter saturated solution of calcium hypochlorite to remove metal carbonyls, if formed. The reactor was pressurized three times with nitrogen, 6,2 bar (90 psig) and vented through the same system.

The residual reactor contents were dumped into a chilled pressure bottle and sealed. Subsequent analysis was performed using a Hewlett-Packard Model 5880 gas chromatograph equipped with a 3,2 mm (one-eighth inch) diameter by 3 m (ten feet) long column packed with Chromosorb 101.

The following examples serve to further illustrate this invention. In the examples the term "AcAc" means "acetylacetonate". Values given for acetic acid obtained include acetic acid equivalents present as methayl acetate.

<u>Control Experiment A</u>

In this experiment the system contained rhodium atom and lithium iodide only.

The autoclave was charged with 1.03 g of $Rh(CO)_2$ AcAc (4 mmoles), 4.28 g of lithium iodide

0158720

(32 mmoles) and 146 g of methyl formate (2.45 moles). Following the procedure described above the reaction was carried out at 180°C and a carbon monoxide pressure of 27,6 bar (400 psig) for 3 hours. The major product was 1.28 moles of acetic acid. The calculated rate to acetic acid was 2.8 gmole/l·h and the conversion of methyl formate to acetic acid was 70%.

## Control Experiments Series B

A series of experiments was carried out using the same procedures and conditions employed in Control Experiment A. In this series the concentrations of rhodium, lithium iodide or methyl iodide were varied; in all instances 146g of methyl formate were charged.

| Run | a | b | c | d | e |
|---|---|---|---|---|---|
| Rh, mmoles | 1 | 2 | 4 | 8 | 8 |
| LiI, mmoles | 64 | 64 | 16 | 0 | 0 |
| $CH_3I$, mmoles | 0 | 0 | 0 | 32 | 120 |
| Rate, gmoles/l·h | 1.5 | 2.8 | 0.5 | 0 | 2.5 |
| Conversion, % | 39 | 70 | 22 | 0 | 60 |

## Example 1

In this example, illustrative of the invention, the autoclave was charged with 1.03 g of $Rh(CO)_2$ AcAc (4 mmoles), 2.14 g of lithium iodide (16 mmoles), 2.27 g of methyl iodide (16 mmoles), 120 ml of methyl formate (1.94 moles) and 30 ml of acetic acid as solvent. The procedure followed was the same as described in Control Experiment A but due to the increased reaction rate the reaction was run for only one hour. The major product was 0.47

mole of acetic acid. The calculated rate to acetic acid was 3.1 gmole/l·h and the conversion of methyl formate to acetic acid was 26%.

Comparison to "Run C" of Control Experiments Series B shows the unpredictable and unexpected increase in rate resulting from the addition of methyl iodide to the reaction; a six-fold increase, accompanied by a 18% increase in conversion.

Example 2

A series of runs was carried out using the same conditions described in Example 1 but varying the concentrations of rhodium, lithium iodide and methyl iodide. The unexpected and unpredictable increases in rate and conversion are obvious upon comparison with the results achieved in the Control Experiments, particular attention should be made to the results of "Run i" with the results of Control Experiment A.

| Run | i | ii | iii |
|---|---|---|---|
| Rh, mmoles | 4 | 4 | 1 |
| LiI, mmoles | 32 | 64 | 96 |
| $CH_3I$, mmoles | 32 | 64 | 32 |
| Reaction Time, h | 0.5 | 0.5 | 1 |
| Rate, gmoles/l·h | 9.9 | 20.1 | 12.6 |
| Conversion, % | 50 | 83 | 100 |

An unpredictable, unexpected and significant rate increase was achieved through the use of mixtures of lithium iodide plus methyl iodide. This was surprising since "Run d" of Control Experiments Series B, which used 32 mmoles of $CH_3I$ with Rh failed to achieve any reaction to

acetic acid. In "Run e" 120 mmoles of $CH_3I$ were required to effect the rate achieved by 64 mmoles in "Run b" at 1/4 the rhodium catalyst charged. The control experiments appeared to indicate that $CH_3I$ was a poor promoter. Thus, it was unpredictable and unexpected to discover the synergism between lithium iodide and methyl iodide with the rhodium catalyst.

CLAIMS

1. A process for the production of organic carboxylic acids of the formula RCOOH which comprises the catalytic reaction of organic formate esters of the formula HCOOR in contact with carbon monoxide and a homogeneous catalyst system consisting essentially of rhodium metal atom and a mixture of lithium iodide and methyl iodide, wherein R is an alkyl group having from 1 to 30 carbon atoms, or aryl, aralkyl or alkaryl groups having 6 or 10 ring carbon atoms with from 1 to 10 carbon atoms in the alk-moiety thereof, or alkenyl having from 2 to 30 carbon atoms.

2. A process as claimed in claim 1 wherein the temperature is from 50°C to 350°C and the pressure is from 10,35 to 690 bar.

3. A process as claimed in claim 1 wherein the mole ratio of Rh:LiI is from 1:1 to 1:1,000 or from 1:8 to 1:150.

4. A process as claimed in claims 1 to 3 wherein the organic formate ester has (i) alkyl groups having from 1 to 15 carbon atoms, (ii) alkenyl groups having from 2 to 15 carbon atoms, or (iii) aryl, aralkyl or alkaryl groups having from 6 to 10 ring carbon atoms and from 1 to 4 carbon atoms in the alk-moiety thereof.

5. A process as claimed in claims 1 to 4 wherein said organic ester is methyl formate.

6. A process as claimed in claims 1 to 5 wherein an organic ligand of the formula $ER_3''$ is present, wherein E is nitrogen, phosphhorus, arsenic, antimony and bismuth and R" is an organic moiety.

7. A process as claimed in claim 6 wherein said ligand is a tertiary amine or a phosphine.

8. A process as claimed in claims 1 to 7 wherein the mole ratio of $LiI:CH_3I$ is from 1:1,000 to 1,000:1 or from 1:450 to 450:1 or from 1:150 to 150:1.

European Patent Office

# EUROPEAN SEARCH REPORT

0158720
Application number

EP 84 11 4574

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 845 121 (EUBANKS et al.)<br>* Claims 1-3,5; column 3, lines 40-55 and line 70 - column 4, line 13, lines 50-54 * | 1,2,4,5 | C 07 C 51/353<br>C 07 C 53/08 //<br>B 01 J 23/58 |
| E | EP-A-0 097 978 (SHELL)<br>* Claims 1,2,9,10,13; page 5, lines 15-25 * | 1,2,4-6,8 | |
| D,Y | GB-A-1 286 224 (AJINOMOTO)<br>* Claims 1-9, 25-27 * | 1,2,4,5 | |
| D,Y | DE-A-3 046 899 (MITSUBISHI GAS)<br>* Claim; page 2, line 32 - page 3, line 3; page 5, line 34 - page 6, line 30; page 7, lines 5-9; page 10, lines 20-34; page 11, lines 30-32; page 12, lines 6-12; page 15, example 5 * | 1,2,4-8 | TECHNICAL FIELDS SEARCHED (Int. Cl.4)<br>C 07 C 51/00<br>C 07 C 53/00<br>C 07 C 67/00<br>C 07 C 69/00 |
| A | US-A-3 689 533 (SCHULTZ)<br>* Claims 1-7; column 5, lines 21-56; column 8, lines 29-50 * | 1,2,6 | |
| A | DE-A-2 236 439 (MONSANTO)<br>* Claims 1-5,8; page 6, last paragraph - page 7, line 13 * | 1,6,8 | |

The present search report has been drawn up for all claims

| Place of search | Date of completion of the search | Examiner |
|---|---|---|
| THE HAGUE | 25-06-1985 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document